(19) Europäisches Patentamt
European Patent Office
Office européen des brevets

(11) Numéro de publication : **0 465 379 A1**

(12) **DEMANDE DE BREVET EUROPEEN**

(21) Numéro de dépôt : **91420196.7**

(51) Int. Cl.$^5$ : **C07D 211/58,** C08K 5/3435

(22) Date de dépôt : **14.06.91**

(30) Priorité : **03.07.90 FR 9008659**

(43) Date de publication de la demande : **08.01.92 Bulletin 92/02**

(84) Etats contractants désignés : **AT BE CH DE DK ES FR GB GR IT LI LU NL SE**

(71) Demandeur : **RHONE-POULENC CHIMIE**
**25, quai Paul Doumer**
**F-92408 Courbevoie Cédex (FR)**

(72) Inventeur : **Gay, Michel**
**10, rue Henri Rolland**
**F-69100 Villeurbanne (FR)**
Inventeur : **Lavault, Sylvie**
**17, rue Jassron**
**F-69003 Lyon (FR)**

(74) Mandataire : **Vignally, Noel et al**
**Rhône-Poulenc Chimie Service Brevets**
**Chimie Centre de Recherches des Carrières**
**B.P. 62**
**F-69192 Saint-Fons Cédex (FR)**

(54) **Nouveaux composés à fonction pipéridinyle et leur utilisation dans les polymères.**

(57) La présente invention concerne de nouveaux composés à fonction pipéridinyle substituée, (I) plus particulièrement tétraméthyl-2,2,6,6 pipéridinyle.

Plus précisément la présente invention concerne de nouveaux polycarbonates organiques à fonction tétraméthyl-2,2,6,6 pipéridinyle, de synthèse relativement simple et efficace pour la protection des polymères organiques, notamment contre la dégradation due à la lumière et plus particulièrement due au rayonnement ultra-violet.

$$R-O-CO-\left[O-Z-O-CO\right]_n-O-R$$

dans laquelle
- Z'représente :
. un radical de formule (III)

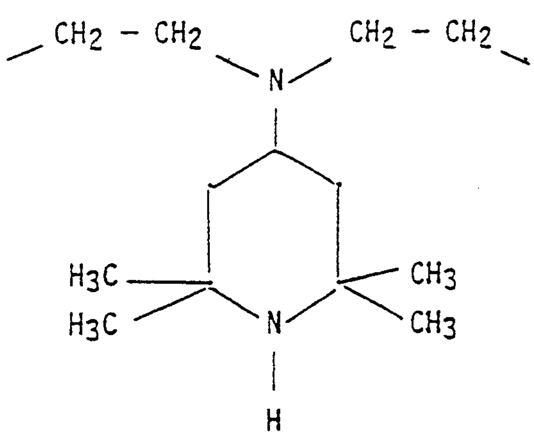

(III)

EP 0 465 379 A1

La présente invention concerne de nouveaux composés à fonction pipéridinyle substituée, plus particulièrement tétraméthyl-2,2,6,6 pipéridinyle.

Elle concerne également l'utilisation de tels composés comme additifs des polymères organiques.

Plus précisément la présente invention concerne de nouveaux composés à fonction tétraméthyl-2,2,6,6 pipéridinyle, de synthèse relativement simple et efficace pour la protection des polymères, notamment contre la dégradation due à la lumière et plus particulièrement due au rayonnement ultra-violet.

Il s'agit de composés à fonction tétraméthyl-2,2,6,6 pipéridinyle, caractérisés en ce qu'ils répondent à la formule générale (I) :

$$R - O - CO - \left[ O - Z - O - CO \right]_n - O - R \qquad (I)$$

dans laquelle :

– R représente :

. un radical alkyle, linéaire ou ramifié, ayant 1 à 18 atomes de carbone,

. un radical cycloalkyle ayant 5 à 12 atomes de carbone,

. un radical phényle,

. un radical phényle comportant 1 ou 2 substituants alkyles ayant 1 à 12 atomes de carbone,

. un radical phénylalkyle dont la partie alkyle, linéaire ou ramifiée, comporte 1 à 12 atomes de carbone,

. un radical tétraméthyl-2,2,6,6 pipéridinyl-4 de formule (II)

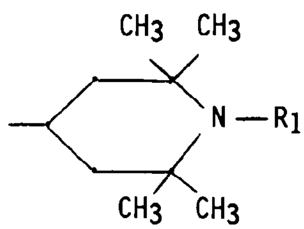

(II)

dans laquelle $R_1$ représente un atome d'hydrogène ou un radical méthyle,

– Z représente :

. un radical de formule (III)

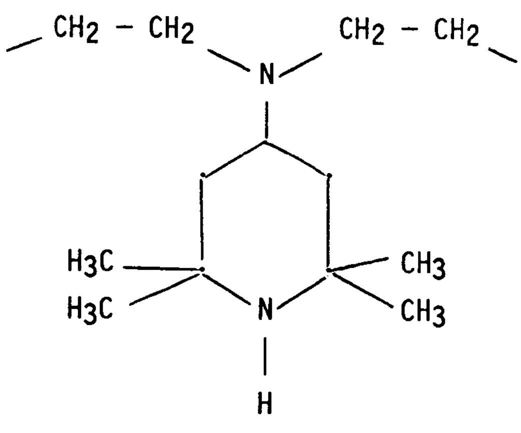

(III)

– n représente un nombre de 1 à 100.

Les composés de l'invention sont plus particulièrement les composés de formule générale (I) dans laquelle :

– R représente :

. un radical alkyle, linéaire ou ramifié, ayant de 1 à 18 atomes de carbone, tel que par exemple les radicaux méthyle, éthyle, propyle, isopropyle, butyle, tertiobutyle, hexyle, octyle, décyle, dodécyle, hexadécyle et octadécyle,

. un radical cyclohexyle,

. un radical phényle,

. un radical phényle comportant 1 à 2 substituants alkyles, linéaires ou ramifiés, ayant 1 à 9 atomes de carbone, tel que par exemple un radical méthyl-phényle, un radical diméthyl-phényle, un radical isopropyl-phényle, un radical diisopropyl-phényle, un radical tertiobutyl-phényle, un radical ditertiobutylphényle, un radical nonyl-phényle, un radical dinonylphényle,

. un radical phénylalkyle dont la partie alkyle, linéaire ou ramifié, comporte 1 à 4 atomes de carbone, tel qu'un radical benzyle, phénéthyle, phényl-3 propyle,

. un radical tétraméthyl-2,2,6,6 pipéridinyl-4 de formule (II) dans laquelle $R_1$ représente un atome d'hydrogène ou un radical méthyle,

– Z représente :

. un radical de formule (III)

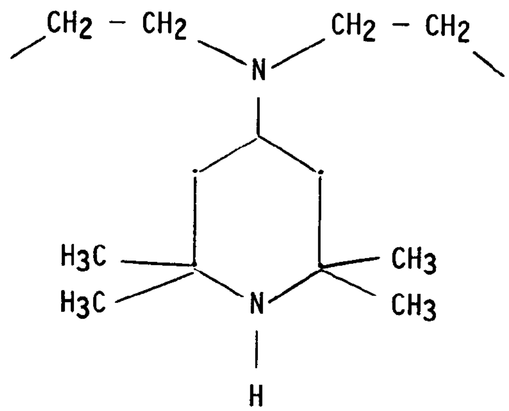

(III)

– n représente un nombre de 1 à 20.

Les composés de formule (I) selon l'invention peuvent être préparés selon divers procédés.

Ainsi lorsque le symbole Z représente un groupement de formule (III) on peut tout d'abord faire réagir le diol de formule générale (IV) :

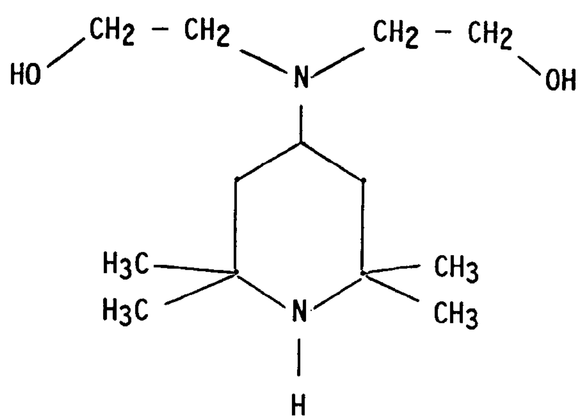

(IV)

avec du carbonate de diméthyle en excès par rapport à la stoechiométrie, en présence d'un catalyseur basique de transestérification tel que par exemple un carbonate de métal alcalin, un alcoolate de métal alcalin, un hydroxyde de métal alcalin et éventuellement en présence d'un agent cryptant tel qu'un éther-couronne.

Le bis-carbonate de diméthyle et de N,N-bis(éthylène)amino-4 tétraméthyl-2,2,6,6 pipéridine de formule (V)

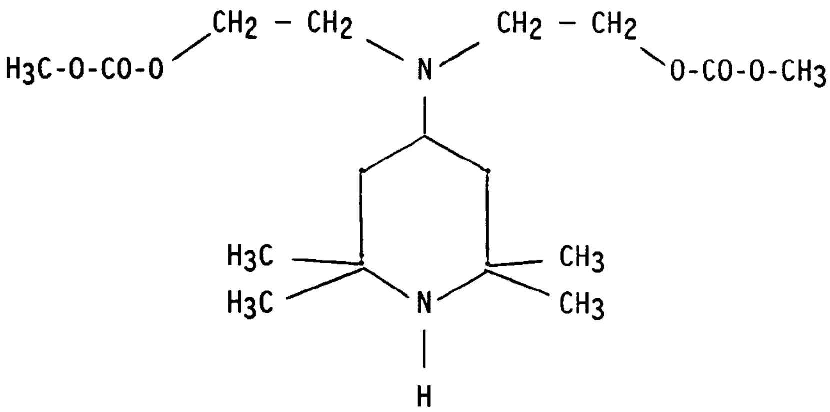

(V)

ainsi obtenu peut être transformé en composé de formule (I) par réaction avec à nouveau le diol (IV) si l'on souhaite un nombre n de motifs supérieurs à 1 et avec un monoalcool ou un monophénol de formule (VI) :

R' - OH

dans laquelle R' a les significations indiquées précédemment pour R dans la formule (I) à l'exception du radical de formule (II).

Les rapports des différents réactifs de formule (V), (IV) et (VI) sont choisis en fonction du nombre n de motifs souhaité : pour $\frac{(n+1)}{2}$ mol de composé (V), on utilise $\frac{(n-1)}{2}$ mol de composé de formule (IV) et 2 mol de composés R' - OH de formule (VI).

Cette réaction est réalisée en présence des mêmes catalyseurs que pour la première réaction.

On peut commodément éliminer à la fin de la première réaction de transestérification l'excès de carbonate de diméthyle, par exemple par distillation sous pression réduite, puis rajouter les composés (IV) et (VI) dans le composé (V) contenant le catalyseur.

Les composés de formule (I) de l'invention peuvent être des composés monomériques ou des composés polymériques selon la valeur du symbole n.

L'invention offre donc un choix important de molécules de masses diverses, ce qui permet d'adapter leurs applications comme anti-UV aux différentes nécessités telles que migration plus facile au sein du polymère pour les objets en masse, permanence dans le temps de l'effet stabilisant dans les films minces ...

Les composés de formule (I) peuvent être utilisés notamment comme stabilisants lumière dans les polymères organiques.

Ainsi ils peuvent être utilisés comme anti-UV dans les polyoléfines, les polystyrènes, les polyalcadiènes, les polyuréthannes, les polyamides, les polyesters, les polycarbonates, les polysulfones, les polyéthers-sulfones, les polyéthers-cétones, les polymères acryliques, les polymères halogénés, leurs copolymères et leurs mélanges.

Les composés de formule (I) sont plus particulièrement utilisés dans les polyoléfines et les polyalcadiènes tels que le polypropylène, le polyéthylène haute densité, le polyéthylène basse densité, le polyéthylène basse densité linéaire, le polybutadiène, leurs copolymères ou leurs mélanges.

Un autre objet de la présente invention consiste donc dans des compositions de polymère organique stabilisé contre les effets néfastes de la lumière et des rayons ultra-violets par une quantité efficace d'au moins un composé de formule (I).

Habituellement ces compositions contiennent de 0,004 à 20 milliéquivalents de fonction tétraméthyl-2,2,6,6 pipéridinyle pour 100 g de polymère.

De préférence les compositions polymériques stabilisées selon l'invention contiennent de 0,020 à 4 milliéquivalents de fonction tétraméthyl-2,2,6,6 pipéridinyle pour 100 g de polymère.

A titre indicatif les compositions polymériques stabilisées contiennent de 0,01 % à 5 % en poids de composé de formule (I).

L'addition des composés de formule (I) peut être effectuée pendant ou après la préparation des polymères.

Ces compositions de polymères organiques contenant les composés de formule (I) peuvent contenir en outre les additifs et stabilisants habituellement utilisés tels que :

– les antioxydants comme les monophénols alkylés, les hydroquinones alkylées, les sulfures de diphényle hydroxylé, les alkylidène-bis-phénols, les composés benzyliques, les acylaminophénols, les esters de l'acide (ditertiobutyl-3,5 hydroxy-4 phényl)-3 propionique, les esters de l'acide tertiobutyl-5 hydroxy-4 méthyl-3 phényl)-3 propionique, les esters de l'acide (dicyclohexyl-3,5 hydroxy-4 phényl)-3 propionique, les

amides de l'acide (ditertiobutyl-3,5 hydroxy-4 phényl)-3 propionique ;

– les absorbeurs de rayons ultra-violets et stabilisants à la lumière comme les (hydroxy-2′ phényl)-2 benzotriazoles, les hydroxy-2 benzophénones, les esters d'acide benzoïque éventuellement substitués, les esters acryliques, les composés du nickel, les oxalamides ;

– les désactivants de métaux ;

– les phosphites et phosphonites ;

– les composés destructeurs de peroxydes ;

– les agents de nucléation ;

– les charges et agents de renforcement ;

– les plastifiants ;

– les lubrifiants ;

– les émulsionnants ;

– les pigments ;

– les azurants optiques ;

– les ignifugeants ;

– les antistatiques ;

– les porogènes.

Les compositions de polymères stabilisées peuvent être mises en oeuvre sous les formes les plus variées, par exemple sous forme d'objets moulés, de feuilles, de fibres, de matériaux cellulaires (masses), de profilés ou de produits de revêtement, ou comme feuillogènes (liants) pour peintures, vernis, colles ou ciments.

Les exemples qui suivent illustrent l'invention.

EXEMPLE 1 : Préparation du produit (Ia) : bis-carbonate de diméthyle et de (N,N-diéthylène-amino)-4 tétraméthyl-2,2,6,6 pipéridine

Composé de formule générale (I) dans laquelle

– R = radical méthyle

– n = 1

– Z = radical de formule (III)

Dans un ballon de 500 $cm^3$, équipé d'une agitation centrale, d'une gaine thermométrique, d'une tubulure latérale d'arrivée d'azote et d'une colonne à distiller, on charge :

– 216 g (2,4 mol) de carbonate de diméthyle

– 48,8 g (0,2 mol) de N,N-bis(hydroxyéthyl)amino-4 tétraméthyl-2,2,6,6 pipéridine

– 0,008 g de soude en poudre.

Le mélange réactionnel est chauffé à 92-95°C pendant 5 heures sous courant d'azote : au cours de cette phase on distille (en maintenant un fort taux de reflux) un binaire méthanol/carbonate de diméthyle (entre 63°C et 70°C).

On monte ensuite la température jusqu'à 120°C pendant 2 h, en continuant à distiller la fraction volatile.

On distille au total 175 g.

On élimine ensuite le reste des produits volatiles à 120°C en diminuant progressivement la pression jusqu'à 133 Pa : on obtient ainsi encore 15 g de produits volatils.

On obtient finalement 70,2 g d'une huile visqueuse, légèrement jaune.

Les analyses par spectrométrie IR et résonance magnétique nucléaire (RMN) confirment la structure attendue du produit dont la pureté est supérieure à 95 %.

EXEMPLE 2 : Préparation du produit (Ib) : hexakis-carbonate de diméthyle et de pentakis(N,N-diéthylèneamino-4 tétraméthyl-2,2,6,6 pipéridine)

Composé de formule générale (I) dans laquelle

– R = radical méthyle

– n = 5

– Z = radical de formule (III)

Dans un ballon de 200 $cm^3$, équipé d'une agitation centrale, d'une gaine thermométrique et d'une colonne à distiller, on charge :

– 43,2 g (0,12 mol) du produit (Ia) préparé dans l'exemple 1

– 19,5 g (0,08 mol) de N,N-bis(hydroxyéthyl)amino-4 tétraméthyl-2,2,6,6 pipéridine

– 0,019 g de carbonate de potassium

– 5 microlitres de l'éther-couronne $C_{18/6}$.

Le mélange réactionnel est chauffé à 105°C pendant 4 h 20 sous une pression de 26 600 Pa.

On diminue ensuite progressivement la pression jusqu'à 27 Pa et on maintient la température à 100-105°C pendant 2 h, en éliminant le méthanol au fur et à mesure de sa formation.

On obtient finalement 58,6 g d'une huile visqueuse jaune.

Les analyses par spectrométrie IR et RMN confirment la structure attendue du produit.

EXEMPLE 3 : Préparation du produit (Ic) : bis-carbonate de bis-(tétraméthyl-2,2,6,6 pipéridinyl-4) et de (N,N-diéthylèneamino)-4 tétraméthyl-2,2,6,6 pipéridine

Composé de formule générale (I) dans laquelle

- R = radical de formule (II) avec $R_1$ = H
- n = 1
- Z = radical de formule (III)

Dans un ballon de 200 $cm^3$, équipé d'une agitation centrale, d'une gaine thermométrique et d'une colonne à distiller, on charge :

- 36 g (0,1 mol) du produit (Ia) préparé dans l'exemple 1
- 31,4 g (0,2 mol) de hydroxy-4 tétraméthyl-2,2,6,6 pipéridine
- 0,0165 g de carbonate de potassium
- 5 microlitres de l'éther-couronne $C_{18/6}$.

Le mélange réactionnel est chauffé à 110-115°C pendant 7 h sous atmosphère d'azote, puis pendant 2 h sous une pression de 26 600 Pa.

On diminue ensuite progressivement la pression jusqu'à 665 Pa et on maintient la température à 115°C pendant 30 min.

Pendant ces opérations, on élimine le méthanol au fur et à mesure de sa formation, ainsi que de faibles quantités d'hydroxy-4 tétraméthyl-2,2,6,6 pipéridine.

On termine l'élimination des composés volatils du mélange réactionnel, par un chauffage à 120°C sous 266 Pa pendant 2 h.

On obtient finalement 55 g d'une huile très visqueuse jaune.

L'analyse RMN montre qu'il s'agit d'un mélange correspondant à :

- produit (Ia) de départ : 10 % en moles
- produit (Ic) attendu : 90 % en moles

EXEMPLE 4 : Préparation du produit (Id) : undecakis-carbonate de bis-(tétraméthyl-2,2,6,6 pipéridinyl-4) et de décakis(N,N-diéthylèneamino-4 tétraméthyl-2,2,6,6 pipéridine)

Composé de formule générale (I) dans laquelle

- R = radical de formule (II) avec $R_1$ = H
- n = 10
- Z = radical de formule (III)

Dans un ballon de 200 $cm^3$, équipé d'une agitation centrale, d'une gaine thermométrique et d'une colonne à distiller, on charge :

- 39,6 g (0,11 mol) du produit (Ia) préparé dans l'exemple 1
- 22,0 g (0,09 mol) de N,N-bis(hydroxyéthyl)amino-4 tétraméthyl-2,2,6,6 pipéridine
- 6,28 g (0,04 mol) de hydroxy-4 tétraméthyl-2,2,6,6 pipéridine
- 0,020 g de carbonate de potassium
- 6 microlitres de l'éther-couronne $C_{18/6}$.

Le mélange réactionnel est chauffé à 110-115°C pendant 7 h sous atmosphère d'azote, puis pendant 2 h sous une pression de 26 600 Pa.

On diminue ensuite progressivement la pression jusqu'à 665 Pa et on maintient la température à 115°C pendant 30 min.

Pendant ces opérations, on élimine le méthanol au fur et à mesure de sa formation, ainsi que de faibles quantités d'hydroxy-4 tétraméthyl-2,2,6,6 pipéridine.

On termine l'élimination des composés volatils du mélange réactionnel, par un chauffage à 120°C sous 266 Pa pendant 2 h.

On obtient finalement 52 g d'une huile très visqueuse jaune.

Les analyses par spectrométrie IR et RMN confirment la structure attendue du produit.

EXEMPLE 5 : Préparation du produit (Ie) : undecakis-carbonate de didodécyle et de décakis(N,N-diéthylèneamino-4 tétraméthyl-2,2,6,6 pipéridine)

Composé de formule générale (I) dans laquelle

– R = radical dodécyle
– n = 10
– Z = radical de formule (III)

Dans un ballon de 200 $cm^3$, équipé d'une agitation centrale, d'une gaine thermométrique et d'une colonne à distiller, on charge :

– 49,5 g (0,138 mol) du produit (Ia) préparé dans l'exemple 1
– 27,45 g (0,113 mol) de N,N-bis(hydroxyéthyl)amino-4 tétraméthyl-2,2,6,6 pipéridine
– 9,3 g (0,05 mol) de n-dodécanol
– 0,020 g de carbonate de potassium
– 5 microlitres de l'éther-couronne $C_{18/6}$.

Le mélange réactionnel est chauffé à 105°C pendant 4 h 20 sous une pression de 26 600 Pa.

On diminue ensuite progressivement la pression jusqu'à 27 Pa et on maintient la température à 100-105°C pendant 2 h, en éliminant le méthanol au fur et à mesure de sa formation.

On obtient finalement 81,1 g d'une huile visqueuse jaune.

Les analyses par spectrométrie IR et RMN confirment la structure attendue du produit.

EXEMPLE 6 : Préparation du produit (If) : hexakis-carbonate de didodécyle et de pentakis(N,N-diéthylèneamino-4 tétraméthyl-2,2,6,6 pipéridine)

Composé de formule générale (I) dans laquelle

– R = radical dodécyle
– n = 5
– Z = radical de formule (III)

Dans un ballon de 200 $cm^3$, équipé d'une agitation centrale, d'une gaine thermométrique et d'une colonne à distiller, on charge :

– 54 g (0,15 mol) du produit (Ia) préparé dans l'exemple 1
– 24,4 g (0,10 mol) de N,N-bis(hydroxyéthyl)amino-4 tétraméthyl-2,2,6,6 pipéridine
– 18,6 g (0,10 mol) de n-dodécanol
– 0,020 g de carbonate de potassium
– 5 microlitres de l'éther-couronne $C_{18/6}$.

Le mélange réactionnel est chauffé à 105°C pendant 4 h 20 sous une pression de 26 600 Pa.

On diminue ensuite progressivement la pression jusqu'à 27 Pa et on maintient la température à 100-105°C pendant 2 h, en éliminant le méthanol au fur et à mesure de sa formation.

On obtient finalement 91,3 g d'une huile visqueuse jaune.

Les analyses par spectrométrie IR et RMN confirment la structure attendue du produit.

EXEMPLE 7 - Photostabilisation du polypropylène (PP) APPRYL 3030 P commercialisé par BP Chimie

Dans un mélangeur lent, on prépare environ 300 g de chacun des mélanges dont la composition pondérale est indiquée dans le tableau (I) suivant :

| Composition | A | B | C | D | E | F | G | H | J | K | L | M | N | P | Q |
|---|---|---|---|---|---|---|---|---|---|---|---|---|---|---|---|
| PP | 100 | 100 | 100 | 100 | 100 | 100 | 100 | 100 | 100 | 100 | 100 | 100 | 100 | 100 | 100 |
| Stéarate de Ca | 0,1 | 0,1 | 0,1 | 0,1 | 0,1 | 0,1 | 0,1 | 0,1 | 0,1 | 0,1 | 0,1 | 0,1 | 0,1 | 0,1 | 0,1 |
| antioxydant phénolique* | 0,05 | 0,05 | 0,05 | 0,05 | 0,05 | 0,05 | 0,05 | 0,05 | 0,05 | 0,05 | 0,05 | 0,05 | 0,05 | 0,05 | 0,05 |
| phosphite ** | 0,05 | 0,05 | 0,05 | 0,05 | 0,05 | 0,05 | 0,05 | 0,05 | 0,05 | 0,05 | 0,05 | 0,05 | 0,05 | 0,05 | 0,05 |
| anti UV commercial*** CHIMASSORB944 | 0 | 0,15 | 0,30 | 0 | 0 | 0 | 0 | 0 | 0 | 0 | 0 | 0 | 0 | 0 | 0 |
| produit Ia Exemple 1 | 0 | 0 | 0 | 0,15 | 0,30 | 0 | 0 | 0 | 0 | 0 | 0 | 0 | 0 | 0 | 0 |
| produit Ib Exemple 2 | 0 | 0 | 0 | 0 | 0 | 0,15 | 0,30 | 0 | 0 | 0 | 0 | 0 | 0 | 0 | 0 |
| produit Ic Exemple 3 | 0 | 0 | 0 | 0 | 0 | 0 | 0 | 0,15 | 0,30 | 0 | 0 | 0 | 0 | 0 | 0 |
| produit Id Exemple 4 | 0 | 0 | 0 | 0 | 0 | 0 | 0 | 0 | 0 | 0,15 | 0,30 | 0 | 0 | 0 | 0 |
| produit If Exemple 6 | 0 | 0 | 0 | 0 | 0 | 0 | 0 | 0 | 0 | 0 | 0 | 0,15 | 0,30 | 0 | 0 |
| produit Ie Exemple 5 | 0 | 0 | 0 | 0 | 0 | 0 | 0 | 0 | 0 | 0 | 0 | 0 | 0 | 0,15 | 0,30 |

TABLEAU I

* tétra(hydroxy-4 ditertiobutyl-3,5 phényl)-3 propionate de pentaérythrityle
** phosphite de tris (ditertiobutyl-2,4 phényle)
*** CHIMASSORB 944

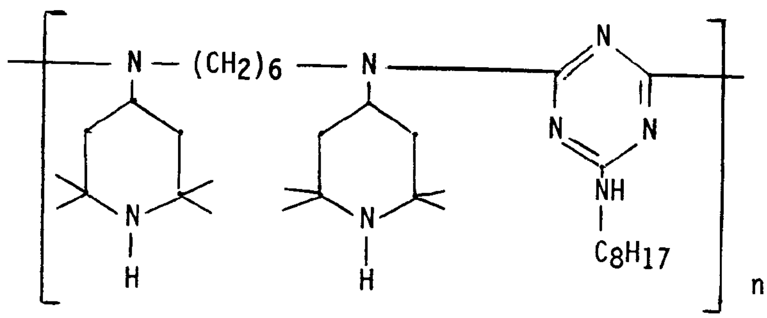

n > 1

Ces compositions sont extrudées dans les conditions suivantes :

– extrudeuse de marque THORET :

diamètre de la vis = 20 mm
longueur de la vis = 400 mm

– profil de température :

. zone 1 = 200°C
. zone 2 = 220°C
. zone 3 = 220°C
. zone 4 = 230°C
. tête de filière = 215°C

Le jonc obtenu est granulé, puis les granulés sont pressés en films de 200 µm à l'aide d'une presse CARVER dans les conditions suivantes :

– température = 210°C
– durée = 5 min
– pression = 20 MPa.

Ces films sont exposés dans une enceinte de vieillissement accéléré de type SAIREM-SEPAP 12-24. Dans cette enceinte les échantillons sont disposés sur une tourelle cylindrique animée d'un mouvement de rotation circulaire. La tourelle est elle-même située au centre d'une enceinte parallélépipédique dont les 4 angles sont occupés par une lampe à vapeur de mercure "moyenne pression" de type MAZDA MA 400 W.

L'enveloppe de la lampe ne laisse passer que les radiations de longueur d'onde supérieure à 300 nm (un tel dispositif est décrit dans le brevet français 2 430 609).

La température de l'enceinte est maintenue à 60°C par un système de régulation.

Le vieillissement des films est suivi par spectrométrie infra-rouge : la densité optique de la bande carbonyle à 1720-1740 $cm^{-1}$ traduit le degré de photooxydation du matériau polymérique.

Les résultats obtenus, rassemblés dans le tableau II ci-après, indiquent le temps nécessaire pour obtenir une densité optique de 0,3 (= "durée de vie" de la composition).

| Composition | Stabilisant anti-UV | Durée pour obtenir une densité optique de 0,3 |
|---|---|---|
| A | néant | 40 h |
| B | CHIMASSORB 944 | 400 h |
| C | CHIMASSORB 944 | 600 h |
| D | Produit Ia | 320 h |
| E | Produit Ia | 400 h |
| F | Produit Ib | 350 h |
| G | Produit Ib | 470 h |
| H | Produit Ic | 440 h |
| J | Produit Ic | 600 h |
| K | Produit Id | 400 h |
| L | Produit Id | 500 h |
| M | Produit If | 350 h |
| N | Produit If | 440 h |
| P | Produit Ie | 320 h |
| Q | Produit Ie | 510 h |

TABLEAU II

## Revendications

**1 -** Composés à fonction tétraméthyl-2,2,6,6 pipéridinyle, caractérisés en ce qu'ils répondent à la formule générale (I) :

$$R - O - CO \left[ O - Z - O - CO \right]_n O - R \qquad (I)$$

dans laquelle :

– R représente :
  - . un radical alkyle, linéaire ou ramifié, ayant 1 à 18 atomes de carbone,
  - . un radical cycloalkyle ayant 5 à 12 atomes de carbone,
  - . un radical phényle,
  - . un radical phényle comportant 1 ou 2 substituants alkyles ayant 1 à 12 atomes de carbone,
  - . un radical phénylalkyle dont la partie alkyle, linéaire ou ramifiée, comporte 1 à 12 atomes de carbone,
  - . un radical tétraméthyl-2,2,6,6 pipéridinyl-4 de formule (II)

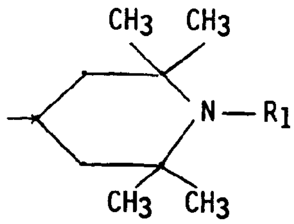

(II)

dans laquelle $R_1$ représente un atome d'hydrogène ou un radical méthyle,

– Z représente :

. un radical de formule (III)

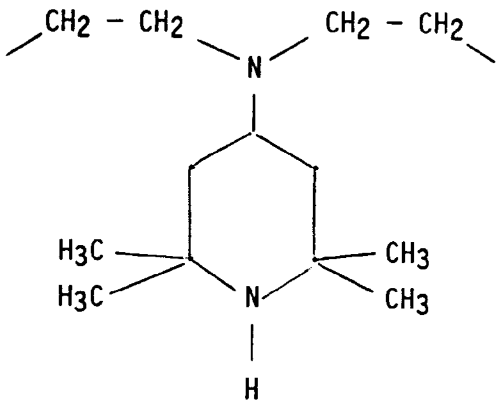

(III)

– n représente un nombre de 1 à 100.

**2 -** Composés selon la revendication 1, caractérisés en ce qu'ils répondent à la formule générale (I) dans laquelle :

– R représente :

. un radical alkyle, linéaire ou ramifié, ayant de 1 à 18 atomes de carbone, tel que par exemple les radicaux méthyle, éthyle, propyle, isopropyle, butyle, tertiobutyle, hexyle, octyle, décyle, dodécyle, hexadécyle, octadécyle,

. un radical cyclohexyle,

. un radical phényle,

. un radical phényle comportant 1 à 2 substituants alkyles, linéaires ou ramifiés, ayant 1 à 9 atomes de carbone, tel que par exemple un radical méthyl-phényle, un radical diméthyl-phényle, un radical isopropyl-phényle, un radical diisopropyl-phényle, un radical tertiobutyl-phényle, un radical ditertiobutylphényle, un radical nonyl-phényle, un radical dinonylphényle,

. un radical phénylalkyle dont la partie alkyle, linéaire ou ramifié, comporte 1 à 4 atomes de carbone, tel qu'un radical benzyle, phénéthyle, phényl-3 propyle,

. un radical tétraméthyl-2,2,6,6 pipéridinyl-4 de formule (II) dans laquelle $R_1$ représente un atome d'hydrogène ou un radical méthyle,

– Z représente :

. un radical de formule (III)

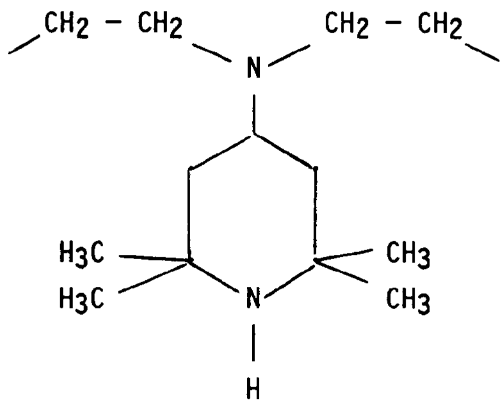

(III)

– n représente un nombre de 1 à 20.

**3 -** Procédé de préparation des composés de formule (I) selon l'une des revendications 1 ou 2, pour lesquels le symbole Z représente un groupement de formule (III), caractérisé en ce que l'on fait réagir le diol de formule générale (IV) :

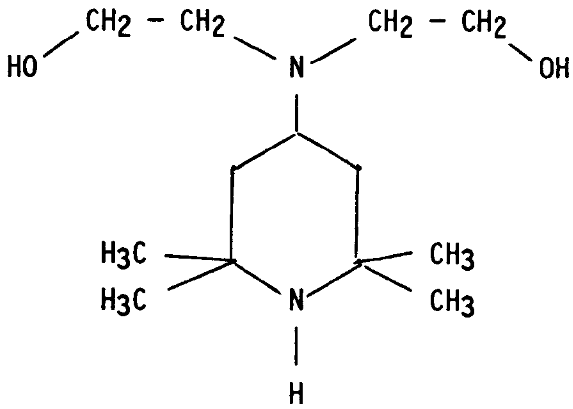

(IV)

avec du carbonate de diméthyle en excès par rapport à la stoechrométrie, en présence d'un catalyseur basique de transestérification et éventuellement en présence d'un agent cryptant ; puis en ce que le bis-carbonate de diméthyle et de N,N-bis(éthylène)amino-4 tétraméthyl-2,2,6,6 pipéridine de formule (V)

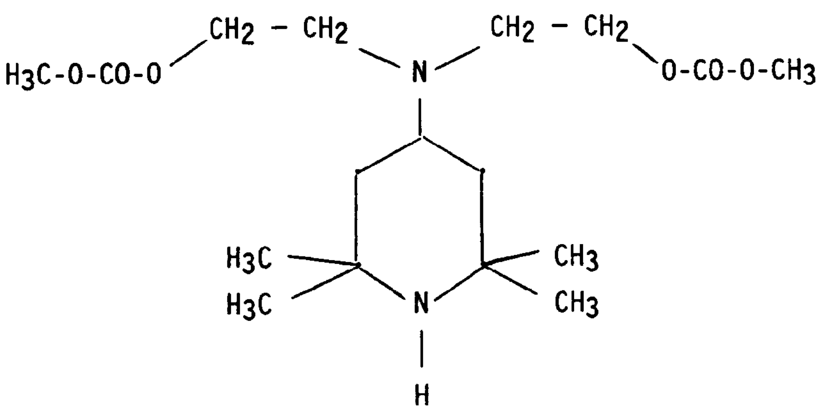

(V)

ainsi obtenu est transformé en composé de formule (I) par réaction avec un monoalcool ou un monophénol de formule (VI) :

R' - OH

dans laquelle R' a les significations indiquées précédemment pour R dans la formule (I) à l'exception du radical de formule (II), et éventuellement avec le diol (IV) si l'on souhaite un nombre n de motifs supérieurs à 1.

**4 -** Procédé selon la revendication 3, caractérisé en ce que les rapports des différents réactifs de formules

(V), (IV) et (VI) sont tels que pour $\frac{(n+1)}{2}$ mol de composé (V), on utilise $\frac{(n-1)}{2}$ mol de composé de formule (IV) et 2 moles de composés R' - OH de formule (VI).

**5 -** Procédé selon l'une des revendications 3 ou 4, caractérisé en ce que l'on élimine à la fin de la première réaction de transestérification l'excès de carbonate de diméthyle, puis que l'on ajoute les composés (IV) et (VI) dans le composé (V) contenant le catalyseur.

**6 -** Compositions de polymère organique stabilisé contre les effets néfastes de la lumière et des rayons ultra-violets par une quantité efficace d'au moins un composé de formule (I), selon l'une des revendications 1 ou 2.

**7 -** Compositions selon la revendication 6, caractérisées en ce que le polymère organique est choisi parmi les polyoléfines, les polystyrènes, les polyalcadiènes, les polyuréthannes, les polyamides, les polyesters, les polycarbonates, les polysulfones, les polyéthers-sulfones, les polyéthers-cétones, les polymères acryliques, les polymères halogénés, leurs copolymères et leurs mélanges.

**8 -** Compositions selon l'une des revendications 6 ou 7, caractérisées en ce que le polymère organique est choisi parmi les polyoléfines et les polyalcadiènes tels que le polypropylène, le polyéthylène haute densité, le polyéthylène basse densité, le polyéthylene basse densité linéaire, le polybutadiène, leurs copolymères ou leurs mélanges.

**9 -** Compositions selon l'une des revendications 6 à 8, caractérisées en ce qu'elles contiennent de 0,004 à 20 milliéquivalents de fonction tétraméthyl-2,2,6,6 pipéridinyle pour 100 g de polymère et de préférence de 0,020 à 4 milliéquivalents de fonction tétraméthyl-2,2,6,6 pipéridinyle pour 100 g de polymère.

Office européen des brevets

# RAPPORT DE RECHERCHE EUROPEENNE

Numero de la demande

EP 91 42 0196

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X,Y | US-A-4 115 476 (MINAGAWA)<br>* En entier *<br>--- | 1-9 | C 07 D 211/58<br>C 08 K 5/3435 |
| Y | EP-A-0 119 961 (CIBA)<br>* En entier; abrégé; page 5 *<br>--- | 1-9 | |
| A | EP-A-0 052 579 (CIBA)<br>* En entier *<br>--- | 1-9 | |
| A | EP-A-0 031 304 (CHIMOSA)<br>* En entier; exemple 7 *<br>--- | 1-9 | |
| A | EP-A-0 094 048 (ARGUS)<br>* En entier *<br>--- | 1-9 | |
| A | US-A-4 124 564 (ARGUS)<br>* En entier *<br>----- | 1-9 | |
| | | | **DOMAINES TECHNIQUES RECHERCHES (Int. Cl.5)** |
| | | | C 07 D 211/00 |

Le présent rapport a été établi pour toutes les revendications

| Lieu de la recherche | Date d'achèvement de la recherche | Examinateur |
|---|---|---|
| LA HAYE | 29-07-1991 | KISSLER B.E. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P0402)